# EUROPÄISCHE PATENTSCHRIFT

(11) **EP 3 094 297 B1**
(45) Veröffentlichungstag und Bekanntmachung des Hinweises auf die Patenterteilung: **27.09.2017**
(21) Anmeldenummer: 15703735.9
(22) Anmeldetag: 15.01.2015
(51) Int. Cl.: A61G 13/02, F16H 19/04, A61B 6/04

(54) **VORRICHTUNG ZUM LINEAREN VERSCHIEBEN EINER PATIENTENLAGERFLÄCHE MIT HILFE EINES HYDRAULIKZYLINDERS UND EINER ZAHNRADANORDNUNG**
DEVICE FOR LINEARLY MOVING A PATIENT SUPPORT SURFACE USING A HYDRAULIC CYLINDER AND A GEAR ARRANGEMENT
DISPOSITIF DE DÉPLACEMENT LINÉAIRE D'UNE SURFACE DE SUPPORT DE PATIENT À L'AIDE D'UN VÉRIN HYDRAULIQUE ET D'UN ENSEMBLE DE ROUES DENTÉES

(30) Priorität: 16.01.2014 DE 102014100444
(43) Veröffentlichungstag der Anmeldung: 23.11.2016
(73) Patentinhaber: Maquet GmbH, 76437 Rastatt (DE)
(72) Erfinder: STAUDINGER, Martin, 76337 Waldbronn (DE); KOCH, Guido, 76149 Karlsruhe (DE)
(74) Vertreter: Zacco GmbH
(86) Internationale Anmeldenummer: PCT/EP2015/050659
(87) Internationale Veröffentlichungsnummer: WO 2015/107107

(56) Entgegenhaltungen:
- WO-A1-02/098342
- WO-A2-2009/047279
- DE-A1- 3 621 480
- US-A1- 2011 083 273
- US-B1- 6 507 964

## Beschreibung

Die Erfindung betrifft eine Vorrichtung zum linearen Verschieben einer Patientenlagereinheit, die eine stationäre Säulenkopfeinheit und eine relativ zur Säulenkopfeinheit linear verschiebbare Lagerflächeneinheit, an der die Patientenlagerfläche befestigbar ist, umfasst. Ferner hat die Vorrichtung einen Hydraulikzylinder zum Verschieben der Lagerflächeneinheit relativ zur Säulenkopfeinheit.

Die WO 02/098342 A1 offenbart eine Vorrichtung zur Positionierung einer Patientenlagerfläche.

Gängige Operationstische bestehen aus einem Operationstischfuß, einer höhenverstellbaren Säule sowie einer Patientenlagerfläche, auf der der zu operierende Patient gelagert wird. Um den Patienten während der Operation röntgen zu können, ist es vorteilhaft, wenn die Patientenlagerfläche relativ zur Säule verschoben werden kann, so dass ein durchleuchtbarer Bereich geschaffen wird, in dem beispielsweise ein C-Bogen eines Röntgengerätes geführt werden kann.

Es sind im Wesentlichen drei Verfahren bekannt, die Lagerflächeneinheit, auf der die Patientenlagerfläche angebracht werden kann, relativ zur stationären Säulenkopfeinheit, die an der dem Operationstischfuß abgewandten Seite des Operationstisches angeordnet ist, zu verschieben. Zum einen sind Operationstische bekannt, bei denen die Lagerflächeneinheit manuell relativ zur stationären Säulenkopfeinheit verschoben werden kann. Dies hat den Nachteil, dass hierfür manuell entsprechende Kräfte aufgebracht werden müssen und somit der Bedienkomfort geschmälert wird. Darüber hinaus ist ein genaues zielgerichtetes Verfahren nur schwer möglich. Um diese Nachteile zu vermeiden, wird bei einer alternativen Ausführungsform die Lagerflächeneinheit über einen Elektromotor relativ zur Säulenkopfeinheit verschoben.

Des Weiteren kann die Lagerflächeneinheit relativ zur Säulenkopfeinheit hydraulisch mit Hilfe eines oder mehrerer Hydraulikzylinder bewegt werden. Das Problem solcher hydraulischen Lösungen ist es, dass der Zylinder länger sein muss als der Hub, den er ausführen kann. Somit ergibt sich eine große Baulänge, die im Säulenkopf des Operationstisches in der Regel nicht zur Verfügung steht, so dass das Verschieben über einen einfachen Hydraulikzylinder nur schwer realisierbar ist.

Ferner ist es bekannt, zum Verschieben der Lagerflächeneinheit relativ zur Säulenkopfeinheit zwei Hydraulikzylinder zu verwenden, die derart nebeneinander angeordnet sind, dass beim Betätigen eines Zylinders der andere Zylinder mitbewegt wird, so dass sich deren Hübe zu dem benötigten Gesamthub addieren. Problematisch an dieser Lösung ist jedoch, dass sie sehr teuer ist und in der Breite viel Bauraum benötigt.

Es ist Aufgabe der Erfindung, eine Vorrichtung zum linearen Verschieben einer Patientenlagerfläche mit Hilfe eines Hydraulikzylinders und ein Verfahren zur Montage dieser Vorrichtung anzugeben, die einen einfachen kompakten Aufbau ermöglichen.

Diese Aufgabe wird durch eine Vorrichtung mit den Merkmalen des Anspruchs 1 sowie durch ein Verfahren mit den Merkmalen des unabhängigen Verfahrensanspruchs gelöst. Vorteilhafte Weiterbildungen der Erfindung sind in den abhängigen Ansprüchen angegeben.

Erfindungsgemäß ist an der Säulenkopfeinheit mindestens eine erste Zahnstange und an der Lagerflächeneinheit mindestens eine zweite Zahnstange angebracht. An der Kolbenstange des Hydraulikzylinders, insbesondere an dem dem Zylinderrohr abgewandten Ende der Kolbenstange, ist eine Zahnradanordnung vorgesehen, wobei diese derart mit der ersten Zahnstange in Eingriff ist, dass die Zahnradanordnung bei einer Betätigung des Hydraulikzylinders sowohl auf der ersten Zahnstange als auch auf der zweiten Zahnstange abrollt. Die Zahnanordnung rollt hierbei vorzugsweise auf der ersten und der zweiten Zahnstange in entgegengesetzte Richtungen ab.

Dieses Abrollen der Zahnradanordnung an den an den beiden Einheiten angeordneten Zahnstangen hat den Vorteil, dass der gesamte Verfahrweg, um den die Lagerflächeneinheit relativ zur Säulenkopfeinheit verschoben wird, sich additiv aus den Abrollwegen der Zahnanordnung auf der ersten und der zweiten Zahnstange ergibt. Somit kann eine Verschiebung der Patientenlagerfläche relativ zur Säulenkopfeinheit um eine Strecke erreicht werden, die wesentlich größer ist als der Hub des Hydraulikzylinders, so dass ein Hydraulikzylinder mit einer möglichst kleinen, kompakten Bauform verwendet werden kann und dennoch der benötigte große Verstellweg realisiert werden kann.

Unter dem Abrollen der Zahnradanordnung wird insbesondere verstanden, dass ein Zahnrad oder mehrere Zahnräder der Zahnradanordnung mit der Zahnstange kämmen und über den Eingriff entsprechend gedreht werden.

Bei einer bevorzugten Ausführungsform umfasst die Zahnradanordnung genau ein Zahnrad, das sowohl mit der ersten als auch der zweiten Zahnstange kämmt. In diesem Fall entspricht der Gesamtverfahrweg, um den die Lagerflächeneinheit relativ zur Säulenkopfeinheit bewegt werden kann, genau dem Doppelten des Hubes des Hydraulikzylinders, da das Zahnrad auf beiden Zahnstangen den gleichen Abrollweg hat, nämlich den Abrollweg, der der Länge des Hubes des Hydraulikzylinders entspricht. Es wird insbesondere ein Hydraulikzylinder verwendet, dessen Hub so gewählt ist, dass sich ein Gesamtverfahrweg zwischen 200 mm und 500 mm ergibt.

Bei einer alternativen Ausführungsform kann die Zahnradanordnung auch ein erstes und mindestens ein zweites Zahnrad umfassen, wobei das erste Zahnrad mit der ersten Zahnstange und das zweite Zahnrad mit der zweiten Zahnstange kämmt. Durch das Verhältnis der Größen der beiden Zahnräder zueinander kann somit das Übersetzungsverhältnis zwischen dem Hub des Hydraulikzylinders und dem Gesamtverfahrweg eingestellt werden, so dass zur Realisierung des gleichen Gesamtverfahrweges ein noch kompakterer Hydraulikzylinder mit einem noch kürzeren Hub verwendet werden kann. Das zweite Zahnrad hat hierbei insbesondere einen größeren Durchmesser als das erste Zahnrad.

Bei einer besonders bevorzugten Ausführungsform beträgt das Zähneverhältnis des ersten Zahnrads zum zweiten Zahnrad zwischen 1:1 und 1:4, insbesondere 12/21 oder 12/24.. Hieraus ergibt ein Übersetzungsverhältnis, also ein Wegverhältnis, von 1 zu 2 bis 1 zu 5. Bei einem Zähneverhältnis von 12/21 ergibt sich ein Übersetzungsverhältnis von 1 zu 2,75, so dass z.B. für die Realisierung eines Gesamtverfahrweges von 300 mm nur ein Hydraulikzylinder mit einem Hub von 109 mm verwendet werden muss. Somit wird ein noch kompakterer Aufbau erreicht.

Ferner ist es vorteilhaft, wenn an der Säulenkopfeinheit eine dritte Zahnstange angeordnet ist und wenn die Zahnradanordnung ein drittes Zahnrad umfasst, das mit der dritten Zahnstange kämmt. Die dritte Zahnstange ist insbesondere in einem vorbestimmten Abstand parallel zur zweiten Zahnstange angeordnet. Das erste und das dritte Zahnrad sind vorzugsweise gleich groß ausgebildet. Somit wird ein noch stabilerer Aufbau erreicht, da sich die Zahnradanordnung auf zwei Zahnstangen der Säulenkopfeinheit abstützen kann. Insbesondere ist das zweite Zahnrad, das mit der zweiten Zahnstange kämmt, zwischen dem ersten und dem dritten Zahnrad angeordnet.

Das erste, zweite und/oder dritte Zahnrad sind insbesondere auf einer gemeinsamen Welle drehfest gelagert, wobei diese Welle relativ zur Kolbenstange drehbar an dieser gelagert ist. Somit ergibt sich ein Zahnradpaket aus den drei Zahnrädern, die sich relativ zueinander nicht drehen können. Ist dagegen nur ein Zahnrad vorgesehen, so kann dies wahlweise ebenfalls drehfest auf einer drehbaren Welle oder alternativ auch drehbar auf einer festen Welle gelagert sein.

Die Welle ist insbesondere drehbar an einem an dem dem Zylinderrohr des Hydraulikzylinders abgewandten Ende der Kolbenstange angeordneten Gabelkopf gelagert. Somit wird eine einfache Verbindung zwischen der Zahnradanordnung und der Kolbenstange erreicht, die dennoch ausreichend stabil ausgebildet ist.

Die Säulenkopfeinheit umfasst vorzugsweise eine Befestigungsanordnung zur Befestigung der Säulenkopfeinheit an einer auf dem Boden lagerbaren Säule eines Operationstisches.

Die Lagerflächeneinheit umfasst insbesondere eine Lagerfläche zur Lagerung der Patientenlagerfläche, an der die Patientenlagerfläche vorzugsweise befestigt werden kann. Ferner hat die Lagerflächeneinheit insbesondere mindestens einen seitlich an der Lagerfläche angeordneten Holm, an dem die zweite Zahnstange angeordnet ist. Vorzugsweise ist ein weiterer Holm an der gegenüberliegenden Seite der Lagerfläche angeordnet. Die zweite Zahnstange ist vorzugsweise an der Innenseite des Holms, also derjenigen Seite, die zu einer Säule eines Operationstisches hingewandt wäre, angeordnet. Somit ist die Anordnung aus den Zahnstangen und dem Hydraulikzylinder verdeckt angebracht, so dass Beschädigungen von außen vermieden werden.

Ferner ist es vorteilhaft, wenn die Säulenkopfeinheit und die Lagerflächeneinheit über mindestens eine Linearführungseinheit, vorzugsweise über mindestens zwei an entgegengesetzten Seiten angeordneten Linearführungseinheiten, miteinander verbunden sind, wobei die Linearführungseinheiten ein lineares Verschieben der Säulenkopfeinheiten relativ zueinander entlang einer vorbestimmten Achse erlauben und eine Bewegung in eine andere Richtung als die durch die vorbestimmte Achse vorgegebene Richtung unterbinden. Die Linearführungseinheiten umfassen vorzugsweise jeweils Linearführungswagen, die an der Säulenkopfeinheit angeordnet sind, wobei die Linearführungswagen in Richtung der vorbestimmten Achse gerichtete Längsaussparungen aufweisen, die an an der Lagerflächeneinheit vorgesehenen Schienen geführt sind. Hierdurch wird eine sichere einfache Führung der Verschiebung erreicht und dennoch eine zuverlässige Lagerung sichergestellt.

Der Hydraulikzylinder ist vorzugsweise an der Säulenkopfeinheit befestigt, was den Vorteil hat, dass er beim Verschieben der Lagerflächeneinheit relativ zur Säulenkopfeinheit nicht mitbewegt werden muss. Insbesondere wird ein Teil des Zylinderbodens in einer Aussparung eines Rahmens der Säulenkopfeinheit aufgenommen, wodurch eine besonders einfache Lagerung erreicht wird. Alternativ kann der Hydraulikzylinder auch an der Lagerflächeneinheit befestigt sein.

Der Hydraulikzylinder umfasst vorzugsweise ein Zylinderrohr, einen Zylinderboden, einen innerhalb des Zylinders bewegbaren Kolben und eine an dem Kolben befestigte Kolbenstange. Die Zahnradanordnung ist an dem dem Kolben entgegengesetzten Ende der Kolbenstange angeordnet. Somit wird ein besonders einfacher Aufbau des Hydraulikzylinders erreicht, so dass kein teurer Sonderbau-Zylinder zugekauft werden muss.

Ein weiterer Aspekt der Erfindung betrifft ein Verfahren zur Montage einer Vorrichtung der zuvor beschriebenen Art, bei dem der Hydraulikzylinder montiert wird, indem zunächst ein Zylinderboden in ein Zylinderrohr eingeführt wird, bis er vollständig in diesem aufgenommen ist. Anschließend werden ein Kolben, eine Kolbenstange und Dichtungselemente in das Zylinderrohr eingeführt. Die so erhaltene Einheit aus dem Zylinderboden, dem Zylinderrohr, dem Kolben, der Kolbenstange und den Dichtungselementen wird in Aufnahmeelemente eines Rahmens der Säulenkopfeinheit eingesetzt. Anschließend wird die Kopfseite des Zylinderrohres, aus der die Kolbenstange hinausragt, mit einem Druck beaufschlagt, so dass ein Teil des Zylinderbodens aus dem Zylinderrohr hinaus in eine Aufnahmebohrung des Rahmens gedrückt wird. Abschließend kann die Ölleitung an der Bodenseite des Hydraulikzylinders angeschlossen werden. An dem dem Zylinderrohr abgewandten Ende der Kolbenstange wird die Zahnradanordnung befestigt.

Das zuvor beschriebene Montageverfahren und die Tatsache, dass lediglich ein Zylinder mit einem geraden Hub benötigt wird, ermöglichen es, einen besonders einfachen Zylinderaufbau umzusetzen, welcher mit wenigen Teilen auskommt, bei dem ein einfaches Standardrohr als Zylinderrohr verwendet werden kann. Dies ermöglicht eine besonders einfache Montage und einen besonders einfachen Aufbau. Insbesondere kann auf teure Sonderformen und -größen des Hydraulikzylinders verzichtet werden. Darüber hinaus sind auch aufwendige Verschraubungen, Verbrödelungen und Schweißnähte nicht notwendig.

Weitere Merkmale und Vorteile der Erfindung ergeben sich aus der folgenden Beschreibung, die die Erfindung anhand von Ausführungsbeispielen im Zusammenhang mit den beigefügten Figuren näher erläutert.

Es zeigen:
- Fig.1: eine schematische, perspektivische Darstellung eines Operationstisches;
- Fig.2: eine schematische, perspektivische Darstellung einer Vorrichtung zum linearen Verschieben einer Patientenlagerfläche des Operationstisches nach Fig.1;
- Fig.3: eine schematische, perspektivische Darstellung einer Säulenkopfeinheit der Vorrichtung nach Fig.2;
- Fig.4: eine Draufsicht auf einen Ausschnitt der Vorrichtung in einem ersten Betriebszustand; und
- Fig.5: eine Schnittdarstellung eines Ausschnitts der Vorrichtung nach den Fig.2 und 4 in einem zweiten Betriebszustand.

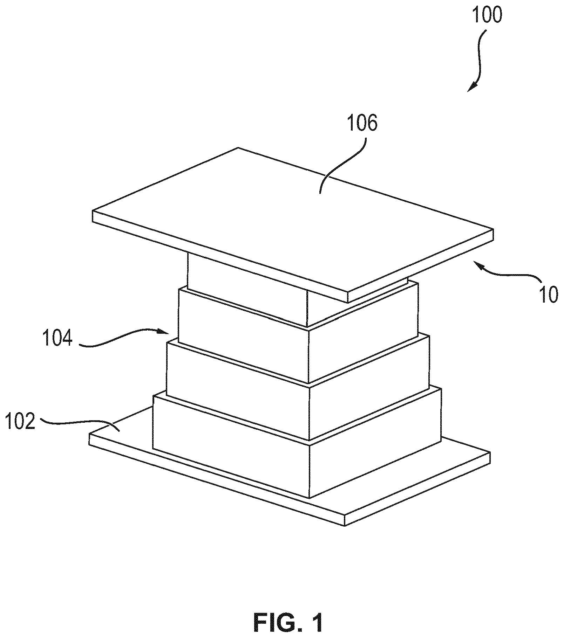

In Fig.1 ist eine schematische, perspektivische stark vereinfachte Darstellung eines Operationstisches 100 gezeigt. Dieser umfasst einen Operationstischfuß 102, über den der Operationstisch 100 auf dem Boden gelagert werden kann. Ferner hat der Operationstisch 100 eine Säule 104, die insbesondere höhenverstellbar ausgebildet ist. An der dem Operationstischfuß 102 abgewandten Seite der Säule 104 ist eine Patientenlagerfläche 106 angeordnet, auf der der Patient gelagert werden kann.

Die Patientenlagerfläche 106 ist mit der Säule 104 über eine Vorrichtung 10 zum linearen Verschieben der Patientenlagerfläche verbunden, so dass die Patientenlagerfläche 106 relativ zur Säule 104 in eine vorbestimmte Richtung entlang einer vorbestimmten Achse linear verschoben werden kann. Dies ist insbesondere dafür notwendig, dass ein möglichst großer durchleuchtbarer Bereich geschaffen wird, innerhalb dessen ein auf der Patientenlagerfläche 106 aufliegender Patient während einer Operation geröntgt werden kann.

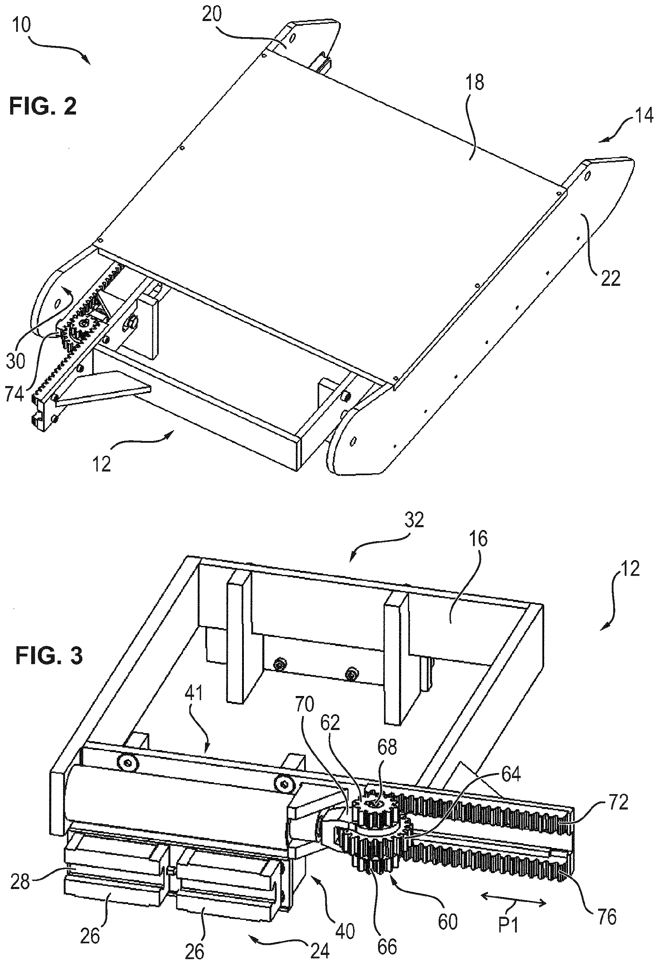

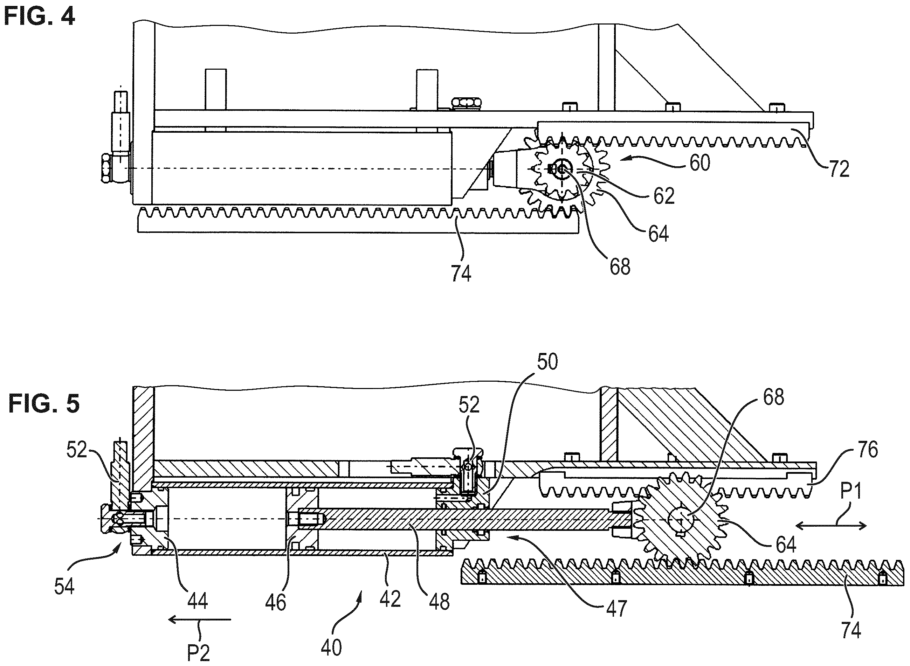

In Fig.2 ist eine schematische, perspektivische Darstellung der Vorrichtung 10 zum linearen Verschieben der Patientenlagerfläche 106 relativ zur Säule 104 dargestellt. Fig.3 zeigt eine schematische, perspektivische Darstellung einer Säulenkopfeinheit 12 der Vorrichtung 10, wobei die Fig.4 und 5 jeweils einen Ausschnitt der Vorrichtung nach Fig.2 in zwei unterschiedlichen Betriebszuständen darstellen, in denen eine Patientenlagereinheit 14 der Vorrichtung 10 relativ zur Säulenkopfeinheit 12 unterschiedlich verschoben ist.

Die Vorrichtung 10 umfasst eine Säulenkopfeinheit 12, über die die Vorrichtung 10 an der Säule 104 des Operationstisches 100 befestigt werden kann. Insbesondere hat die Säulenkopfeinheit 12 einen Rahmen 16, über den die Befestigung möglich ist und der der Säulenkopfeinheit 12 die nötige Stabilität verleiht.

Darüber hinaus hat die Vorrichtung 10 eine Patientenlagereinheit 14, die eine Lagerfläche 18 und zwei seitliche Holme 20, 22 umfasst. Die Patientenlagereinheit 106 ist auf der Lagerfläche 18 befestigbar und wird von dieser abgestützt. Wird also die Lagerflächeneinheit 14 relativ zur Säulenkopfeinheit 12 verschoben, so wird auch die Patientenlagerfläche 106 relativ zur Säule 104 entsprechend verschoben.

Die Säulenkopfeinheit 12 und die Patientenlagerfläche 14 sind über eine Linearführungseinheit 24 in Richtung des Doppelpfeiles P1 relativ zueinander verschiebbar miteinander verbunden. Diese Linearführungseinheit 24 umfasst zwei Führungswagen 26, die jeweils eine in Richtung des Doppelpfeiles P1 verlaufende längliche Aussparung 28 aufweisen, über die sie auf einer nicht gezeigten Schiene geführt sind, die an der Innenseite 30 des Holmes 20 angeordnet ist.

Insbesondere ist eine zweite Linearführungseinheit 32 an der der ersten Linearführungseinheit 24 gegenüberliegenden Seite der Säulenkopfeinheit 12 angeordnet, so dass die Lagerflächeneinheit 14 und die Säulenkopfeinheit 12 beidseitig stabil miteinander verbunden sind. Über die Linearführungseinheit 24, 32 wird erreicht, dass die Lagerflächeneinheit 14 in die durch die Linearführungseinheit 24, 32 vorgegebene Richtung linear verschiebbar an der Säulenkopfeinheit 14 gelagert ist, und in alle anderen Richtungen, die von der durch den Doppelpfeil P1 angedeuteten Richtung abweichen, eine sichere Lagerung besteht.

Zum Verschieben der Lagerflächeneinheit 14 relativ zur Säulenkopfeinheit 12 ist ein Hydraulikzylinder 40 vorgesehen, der in einer Aufnahme 41 des Rahmens 16 angeordnet ist. Der Hydraulikzylinder 40 umfasst ein Zylinderrohr 42, einen Zylinderboden 44 sowie einen Kolben 46, der innerhalb des Zylinderrohres 42 in Richtung des Doppelpfeiles P1 bewegbar geführt ist. An dem Kolben 46 ist eine Kolbenstange 48 angeordnet, die an der dem Zylinderboden 44 abgewandten Seite des Hydraulikzylinders 40 aus dem Zylinderrohr 42 hinausragt. Die Kopfseite 47 des Zylinders 40 ist über einen Zylinderkopf 50 abgedichtet, in dem auch die Kolbenstange 48 geführt ist. Sowohl in dem Zylinderboden 44 als auch in dem Zylinderkopf 50 sind jeweils Hydraulikanschlüsse zum Zu- und Abführen von Öl zum Bewegen des Kolbens 46 relativ zum Zylinderrohr 42 vorgesehen.

Der zuvor beschriebene Aufbau ermöglicht es, dass der Zylinder 40 auf einfache Weise aus Standardteilen zusammengesetzt werden kann und keine komplizierten Sonderanfertigungen benötigt werden. Zur Montage wird der Zylinderboden 40 in das Zylinderrohr 42 eingeführt, wobei ein einfaches Standardrohr als Zylinderrohr 42 verwendet werden kann. Anschließend werden der Kolben 46 und die Kolbenstange 48 zusammen mit entsprechenden Dichtungen in das Zylinderrohr 42 eingeführt. Ferner wird der Zylinderkopf 50 angebracht. Anschließend wird die Kopfseite 47 des Hydraulikzylinders 40 mit einem Druck beaufschlagt, so dass der Zylinderboden in Richtung des Pfeiles P2 gedrückt wird, wodurch ein Teil des Zylinderbodens 44 in eine Aussparung 54 des Rahmens 16 aufgenommen wird, so dass der Zylinder 40 an dem Rahmen 16 fixiert wird. Anschließend müssen nur noch die Hydraulikanschlüsse 52 angebracht werden.

An der dem Kolben 46 abgewandten Seite des Kolbens 48 ist eine Zahnradanordnung 60 vorgesehen, die ein erstes Zahnrad 62, ein zweites Zahnrad 64 und ein drittes Zahnrad 66 umfasst, die alle drehfest auf einer gemeinsamen Welle 68 gelagert sind und somit ein Zahnradpaket bilden.

Die Welle 68 wiederum ist an einem fest an dem Kolben 48 befestigten Gabelkopf 70 relativ zu diesem drehbar gelagert. Somit drehen sich die Zahnräder 62 bis 68 immer gleichmäßig mit der Welle 68.

An der Säulenkopfeinheit 12 sind eine erste Zahnstange 72 und eine dritte Zahnstange 76 angeordnet, die in einem vorbestimmten Abstand parallel zueinander verlaufen. An der Innenseite 30 des Holms 20 der Patientenlagerflächeneinheit 14 ist eine zweite Zahnstange 74 angeordnet, die vorzugsweise parallel zu der ersten und der dritten Zahnstange 72, 76 verläuft.

Das erste Zahnrad 62 kämmt mit der ersten Zahnstange 72, das zweite Zahnrad 64 mit der zweiten Zahnstange 74 und das dritte Zahnrad 66 mit der dritten Zahnstange 76.

In Fig.4 ist der Betriebszustand dargestellt, bei dem die Kolbenstange 48 maximal im Zylinderrohr 42 aufgenommen ist. Wird der Hydraulikzylinder 40 betätigt, so wird die Kolbenstange 48 aus dem Zylinderrohr 42 bewegt, wodurch die Lagerflächeneinheit 14 relativ zur Säulenkopfeinheit 12 verschoben wird.

Hierbei rollen das erste und das dritte Zahnrad 62, 66 auf der ersten und dritten Zahnstange 72, 76 in entgegengesetzte Richtung wie das zweite Zahnrad 64 auf der zweiten Zahnstange 74 ab. Hierdurch wird erreicht, dass sich die Abrollwege zu dem Verfahrweg, um den die Lagerflächeneinheit 14 relativ zur Säulenkopfeinheit 12 verfahren wird, addieren. Dies hat den Vorteil, dass mit Hilfe eines verhältnismäßig kleinen Hubes ein verhältnismäßig großer Gesamtverfahrweg realisiert werden kann, so dass ein kleiner, kompakt und einfach aufgebauter Hydraulikzylinder 40 verwendet werden kann, der problemlos in die Rahmenkonstruktion 16 des Säulenkopfes 12 integriert werden kann.

Bei dem gezeigten Ausführungsbeispiel beträgt das Zähneverhältnis des ersten Zahnrades 62 zum zweiten Zahnrad 64, und somit auch Zähneverhältnis des dritten Zahnrades 66 zum zweiten Zahnrad 64 jeweils 12/21, so dass sich ein Übersetzungsverhältnis zwischen dem Hub und dem realisierten Gesamtverfahrweg von 1 zu 2,75 ergibt. Der Gesamtverfahrweg beträgt insbesondere zwischen 200 mm und 500 mm.

Bei einer alternativen Ausführungsform können auch andere Zähneverhältnisse gewählt werden, wodurch sich andere Übersetzungsverhältnisse ergeben und somit das Verhältnis zwischen der Länge des Hydraulikzylinders 40 und dem realisierbaren Gesamtverfahrweg eingestellt werden kann.

Ferner ist es alternativ möglich, dass auf die dritte Zahnstange 76 und somit auch auf das dritte Zahnrad 66 verzichtet wird.

Darüber hinaus kann alternativ der Hydraulikzylinder 40 auch an der Lagerflächeneinheit 14 befestigt werden.

Bei einer weiteren alternativen Ausführungsform ist lediglich ein Zahnrad 62 bis 66 vorgesehen, welches in zwei Schienen 72, 74 kämmt, von denen eine an der Säulenkopfeinheit 12 und eine an der Lagerflächeneinheit 14 angeordnet ist. Bei diesem Ausführungsbeispiel beträgt das Übersetzungsverhältnis 1 zu 2, so dass der Gesamtverfahrweg dem Doppelten des Hubes des Zylinders 40 entspricht. Hierdurch wird ein noch einfacherer und kostengünstiger Aufbau erreicht.

### Bezugszeichenliste

- 10: Vorrichtung
- 12: Säulenkopfeinheit
- 14: Lagerflächeneinheit
- 16: Rahmen
- 18: Lagerfläche
- 20, 22: Holm
- 24: Linearführungseinheit
- 26: Führungswagen
- 28: Aussparung
- 30: Innenseite
- 32: Linearführungseinheit
- 40: Hydraulikzylinder
- 41: Aufnahme
- 42: Zylinderrohr
- 44: Zylinderboden
- 46: Kolben
- 47: Kopfseite
- 48: Kolbenstange
- 50: Zylinderkopf
- 52: Hydraulikanschluss
- 54: Aussparung
- 60: Zahnradanordnung
- 62, 64, 66: Zahnrad
- 68: Welle
- 70: Gabelkopf
- 72, 74, 76: Zahnstange
- 100: Operationstisch
- 102: Operationstischfuß
- 104: Säule
- 106: Patientenlagerfläche
- P1, P2: Richtung

## Patentansprüche

1. Vorrichtung zum linearen Verschieben einer Patientenlagerfläche,
mit einer stationären Säulenkopfeinheit (12),
einer relativ zur Säulenkopfeinheit (12) linear verschiebbaren Lagerflächeneinheit (14), an der die Patientenlagerfläche (106) befestigbar ist, und
mit mindestens einem Hydraulikzylinder (40) zum Verschieben der Lagerflächeneinheit (14) relativ zur Säulenkopfeinheit (12),
**dadurch gekennzeichnet, dass** an der Säulenkopfeinheit (12) mindestens eine erste Zahnstange (72) angebracht ist,
dass an der Lagerflächeneinheit (14) mindestens eine zweite Zahnstange (74) angebracht ist,
dass an der Kolbenstange (48) des Hydraulikzylinders (40) eine Zahnradanordnung (60) angeordnet ist,
und dass die Zahnradanordnung (60) derart mit der ersten Zahnstange (72) und der zweiten Zahnstange (74) in Eingriff ist, dass die Zahnradanordnung (60) bei einer Betätigung des Hydraulikzylinders (40) sowohl auf der ersten Zahnstange (72) als auch der zweiten Zahnstange (74) abrollt.

2. Vorrichtung (10) nach Anspruch 1, **dadurch gekennzeichnet, dass** die Zahnradanordnung (60) auf der ersten Zahnstange (72) und der zweiten Zahnstange (74) in entgegengesetzte Richtung abrollt.

3. Vorrichtung (10) nach Anspruch 1 oder 2, **dadurch gekennzeichnet, dass** die Zahnradanordnung (60) genau ein Zahnrad (62 bis 66) umfasst, das sowohl mit der ersten Zahnstange (72) als auch der zweiten Zahnstange (74) kämmt.

4. Vorrichtung (10) nach Anspruch 1 oder 2, **dadurch gekennzeichnet, dass** die Zahnradanordnung (60) ein erstes Zahnrad (62) und mindestens eine zweites Zahnrad (64) umfasst, dass das erste Zahnrad (62) mit der ersten Zahnstange (72) kämmt, und dass das zweite Zahnrad (64) mit der zweiten Zahnstange (74) kämmt.

5. Vorrichtung (10) nach Anspruch 4, **dadurch gekennzeichnet, dass** das zweite Zahnrad (64) einen größer Durchmesser als das erste Zahnrad (62) hat.

6. Vorrichtung (10) nach Anspruch 4 oder 5, **dadurch gekennzeichnet, dass** das Zähneverhältnis des ersten Zahnrads (62) zum zweiten Zahnrad (64) zwischen 1 zu 1 und 1 zu 4 beträgt.

7. Vorrichtung (10) nach einem der Ansprüche 4 bis 6, **dadurch gekennzeichnet, dass** an der Säulenkopfeinheit (12) eine dritten Zahnstange (76) angeordnet ist, die insbesondere in einem vorbestimmten Abstand parallel zur ersten Zahnstange (72) verläuft, und dass die Zahnradanordnung (60) ein drittes Zahnrad (66) umfasst, das mit der dritten Zahnstange (76) kämmt.

8. Vorrichtung (10) nach einem der Ansprüche 4 bis 7, **dadurch gekennzeichnet, dass** die Zahnräder (62 bis 66) auf einer gemeinsamen Welle (68) drehfest gelagert sind, und dass die Welle (68) relativ zur Kolbenstange (48) drehbar an dieser gelagert ist.

9. Vorrichtung (10) nach Anspruch 8, **dadurch gekennzeichnet, dass** die Welle (68) drehbar an einer an dem dem Zylinderrohr (42) des Hydraulikzylinders (40) abgewandten Ende der Kolbenstange (48) angeordneten Gabelkopf (70) gelagert ist.

10. Vorrichtung (10) nach einem der vorhergehenden Ansprüche, **dadurch gekennzeichnet, dass** die Säulenkopfeinheit (12) eine Befestigungsanordnung (16) zur Befestigung der Säulenkopfeinheit (12) an einer auf dem Boden lagerbaren Säule (104) umfasst.

11. Vorrichtung (10) nach einem der vorhergehenden Ansprüche, **dadurch gekennzeichnet, dass** die Lagerflächeneinheit (14) eine Lagerfläche (18) zur Lagerung der Patientenlagerfläche (106) und mindestens einen seitlich den der Lagerfläche (18) befestigten Holm (20, 22) umfasst, an dem die zweiten Zahnstange (74), insbesondere an einer Innenseite (30), angeordnet ist.

12. Vorrichtung (10) nach einem der vorhergehenden Ansprüche, **dadurch gekennzeichnet, dass** die Säulenkopfeinheit (12) und die Lagerflächeneinheit (14) über mindestens eine Linearführungseinheit (24, 32), vorzugsweise über mindestens zwei an gegenüberliegenden Seiten angeordnete Linearführungseinheiten (24, 32), miteinander verbunden sind, wobei die Linearführungseinheit (24, 32) ein lineares Verschieben der Säulenkopfeinheit (12) und der Lagerflächeneinheit (14) relativ zueinander entlang einer vorbestimmten Achse erlaubt und eine andere Bewegung der Säulenkopfeinheit (12) und der Lagerflächeneinheit (14) zueinander unterbindet.

13. Vorrichtung (10) nach einem der vorhergehenden Ansprüche, **dadurch gekennzeichnet, dass** der Hydraulikzylinder (40), insbesondere sein Zylinderrohr (42) und/oder sein Zylinderboden (44), an der Säulenkopfeinheit (12) befestigt ist.

14. Vorrichtung (10) nach einem der vorhergehenden Ansprüche, **dadurch gekennzeichnet, dass** der Hydraulikzylinder (40) ein Zylinderrohr (42), einen Zylinderboden (44), einen innerhalb des Zylinderrohres (42) bewegbaren Kolben (46) und eine an dem Kolben (46) befestigte Kolbenstange (48) umfasst, und dass die Zahnradanordnung (60) an dem dem Kolben (46) entgegengesetzten Ende der Kolbenstange (48) angeordnet ist.

15. Verfahren zur Montage einer Vorrichtung (10) nach einem der Ansprüche 1 bis 14,
bei dem der Hydraulikzylinder (40) montiert wird, indem zunächst ein Zylinderboden (44) in ein Zylinderrohr (42) eingeführt wird, bis er in diesem vollständig aufgenommen ist,
anschließend ein Kolben (46), eine Kolbenstange (48) und Dichtungselemente (50) in das Zylinderrohr (42) eingeführt werden,
die so erhaltene Einheit in Aufnahmeelemente (41) eines Rahmens (16) der Säulenkopfeinheit (12) eingesetzt wird,
anschließend die Kopfseite (47) des Zylinderrohres (42), aus der die Kolbenstange (48) hinausragt, mit einem Druck beaufschlagt wird, so dass ein Teil des Zylinderbodens (44) aus dem Zylinderrohr (42) hinaus in eine Aufnahmeaussparung (54) des Rahmens (16) gedrückt wird,
und anschließend eine Ölleitung an der Bodenseite des Hydraulikzylinders (40) angeschlossen wird,
und bei dem an dem dem Zylinderrohr (42) abgewandten Ende der Kolbenstange (48) die Zahnradanordnung (60) befestigt wird.

## Claims

1. A device for linearly moving a patient support surface,
having a stationary column head unit (12),
a linearly movable support surface unit (14) with respect to the column head unit (12), at which support surface unit the patient support surface (106) is mountable, and
having at least one hydraulic cylinder (40) to move the support surface unit (14) relative to the column head unit (12),
**characterized in that** at least a first gear rack (72) is affixed at the column head unit (12),
that at least a second gear rack (74) is affixed to the support surface unit (14),
that a gear arrangement (60) is arranged at the piston rod (48) of the hydraulic cylinder (40),
and that the gear arrangement (60) engages with the first gear rack (72) and the second gear rack (74) in such way that the gear arrangement (60), upon actuation of the hydraulic cylinder (40), unwinds both on the first gear rack (72) and the second gear rack (74).

2. The device (10) according to claim 1, **characterized in that** the gear arrangement (60) unwinds on the first gear rack (72) and the second gear rack (74) in opposite direction.

3. The device (10) according to claim 1 or 2, **characterized in that** the gear arrangement (60) comprises exactly one gear (62 to 66), which combs both with the first gear rack (72) and the second gear rack (74).

4. The device (10) according to claim 1 or 2, **characterized in that** the gear arrangement (60) comprises a first gear (62) and at least a second gear (64), that the first gear (62) combs with the first gear rack (72), and that the second gear (64) combs with the second gear rack (74).

5. The device (10) according to claim 4, **characterized in that** the second gear (64) has a larger diameter than the first gear (62).

6. The device (10) according to claim 4 or 5, **characterized in that** the gear ratio of the first gear (62) to the second gear (64) is between 1 : 1 and 1 : 4.

7. The device (10) according to any of the claims 4 to 6, **characterized in that** a third gear rack (76) is arranged at the column head unit (12), which gear rack particularly extends in a predefined distance parallel to the first gear rack (72), and that the gear arrangement (60) comprises a third gear (66), which combs with the third gear rack (76).

8. The device (10) according any of the claims 4 to 7, **characterized in that** the gears (62 to 66) are rotatably fixed on a common shaft (68), and that the shaft (68) is rotatably mounted relative to the piston rod (48) at such rod.

9. The device (10) according to claim 8, **characterized in that** the shaft (68) is rotatably mounted at a fork head (70), which is arranged at the averted end of the piston rod (48) with respect to the cylinder liner (42) of the hydraulic cylinder (40).

10. The device (10) according to any of the preceding claims, **characterized in that** the column head unit (12) comprises a mounting arrangement (16) for mounting the column head unit (12) to a column (104), which is supportable on the floor.

11. The device (10) according to any of the preceding claims, **characterized in that** the support surface unit (14) comprises a support surface (18) for supporting the patient support surface (106) and at least a tie bar (20, 22), which is laterally mounted to the support surface (18), at which bar the second gear rack (74) is arranged, particularly at an inner side (30).

12. The device (10) according to any of the preceding claims, **characterized in that** the column head unit (12) and the support surface unit (14) are connected to one another via at least one linear guide unit (24, 32), preferably via at least two linear guide units (24, 32) arranged at opposing sides, whereby the linear guide unit (24, 32) allows for a linear moving of the column head unit (12) and the support surface unit (14) relative to one another along a predefined axis and prevents another movement of the column head unit (12) and the support surface unit (14) with respect to one another.

13. The device (10) according to any of the preceding claims, **characterized in that** the hydraulic cylinder (40), in particular its cylinder liner (42) and/or its cylinder floor (44), is mounted to the column head unit (12).

14. The device (10) according to any of the preceding claims, **characterized in that** the hydraulic cylinder (40) comprises a cylinder liner (42), a cylinder floor (44), a movable piston (46) within the cylinder liner (42), and a piston rod (48) mounted to the piston (46), and that the gear arrangement (60) is arranged at the end of the piston rod (48), which is opposite to the piston (46).

15. A method for mounting a device (10) according any of the claims 1 to 14,
where the hydraulic cylinder (40) is mounted by first inserting a cylinder floor (44) into a cylinder liner (42), until it is completed received in such liner,
subsequently inserting a piston (46), a piston rod (48) and sealing elements (50) into the cylinder liner (42),
inserting the thus obtained unit into receiving elements (41) of a frame (16) of the column head unit (12),
subsequently applying pressure to the head side (47) of the cylinder liner (42), from which the piston rod (48) protrudes, so that a part of the cylinder floor (44) is pressed out of the cylinder liner (42) into a receiving recess (54) of the frame (16),
and subsequently connecting an oil line to the floor side of the hydraulic cylinder (40),
and mounting the gear arrangement (60) at the end facing away from the cylinder liner (42) of the piston rod (48).

## Revendications

1. Dispositif de déplacement linéaire d'une surface de support de patient
avec une unité de tête de colonne (12) stationnaire,
d'une unité de surface de support (14) déplaçable linéairement par rapport à l'unité de tête de colonne (12) stationnaire, sur laquelle la surface de support de patient (106) peut être fixée, et
doté d'au moins un cylindre hydraulique (40) pour le déplacement de l'unité de surface de support (14) par rapport à l'unité de tête de colonne (12),
**caractérisé en ce qu'**au moins une première crémaillère (72) est fixée sur l'unité de tête de colonne (12),
qu'au moins une seconde crémaillère (74) est fixée sur l'unité de surface de support (14),
qu'un dispositif d'engrenage (60) est disposé sur la tige de piston (48) du cylindre hydraulique (40),
et que le dispositif d'engrenage (60) s'encliquète avec la première crémaillère (72) et la seconde crémaillère (74) de telle sorte que le dispositif d'engrenage (60) se déroule aussi bien sur la première crémaillère (72) que sur la seconde crémaillère (74) lors d'un actionnement du cylindre hydraulique (40).

2. Dispositif (10) selon la revendication 1, **caractérisé en ce que** le dispositif d'engrenage (60) se déroule sur la première crémaillère (72) et sur la seconde crémaillère (74) dans des directions opposées.

3. Dispositif (10) selon la revendication 1 ou 2, **caractérisé en ce que** le dispositif d'engrenage (60) comprend précisément une roue dentée (62 à 66), qui s'engrène aussi bien avec la première crémaillère (72) qu'avec la seconde crémaillère (74).

4. Dispositif (10) selon la revendication 1 ou 2, **caractérisé en ce que** le dispositif d'engrenage (60) comprend une première roue dentée (62) et au moins une seconde roue dentée (64), que la première roue dentée (62) s'engrène avec la première crémaillère (72), et que la seconde roue dentée (64) s'engrène avec la seconde crémaillère (74).

5. Dispositif (10) selon la revendication 4, **caractérisé en ce que** la seconde roue dentée (64) possède un diamètre supérieur à celui de la première roue dentée (62).

6. Dispositif (10) selon la revendication 4 ou 5, **caractérisé en ce que** la valeur du rapport des dents de la première roue dentée (62) sur les dents de la seconde roue dentée (64) est comprise entre 1:1 à 1:4.

7. Dispositif (10) selon l'une quelconque des revendications 4 à 6, **caractérisé en ce qu'**une troisième crémaillère (76) est disposée sur l'unité de tête de colonne (12), qui se déroule en particulier dans un intervalle prédéterminé parallèle à la première crémaillère (72), et que le dispositif d'engrenage (60) comprend une troisième roue dentée (66) qui s'engrène avec la troisième crémaillère (76).

8. Dispositif (10) selon l'une quelconque des revendications 4 à 7, **caractérisé en ce que** les roues d'engrenage (62 à 66) sont installées de manière solidaire sur un arbre commun (68), et que l'arbre (68) est installé de manière mobile sur celui-ci, par rapport à la tige de piston (48).

9. Dispositif (10) selon la revendication 8, **caractérisé en ce que** l'arbre (68) est installé de manière rotative sur une chape (70) disposée sur extrémité de la tige de piston (48) opposée au tube cylindrique (42) du cylindre hydraulique (40).

10. Dispositif (10) selon l'une quelconque des revendications précédentes, **caractérisé en ce que** l'unité de tête de colonne (12) comprend un dispositif de fixation (16) pour fixer l'unité de tête de colonne (12) sur l'une des colonnes (104) pouvant être placée sur le sol.

11. Dispositif (10) selon l'une quelconque des revendications précédentes, **caractérisé en ce que** l'unité de surface de support (14) comprend une surface de support (18) pour le support de la surface de support du patient (106) et au moins une barre de liaison (20, 22) montée latéralement à celles de la surface de support (18), sur laquelle barre la seconde crémaillère (74) est disposée, en particulier sur une face interne (30).

12. Dispositif (10) selon l'une quelconque des revendications précédentes, **caractérisé en ce que** l'unité de tête de colonne (12) et l'unité de surface de support (14) sont reliées entre elles via au moins une unité de guidage linéaire (24, 32), de préférence via au moins deux unités de guidage linéaire (24, 32) disposées sur des côtés opposés, dans lequel l'unité de guidage linéaire (24, 32) permet un déplacement linéaire de l'unité de tête de colonne (12) et de l'unité de surface de support (14) relativement l'une par rapport à l'autre le long d'un axe prédéterminé et empêche un autre mouvement de l'unité de tête de colonne (12) et de l'unité de surface de support (14) l'une par rapport à l'autre.

13. Dispositif (10) selon l'une quelconque des revendications précédentes, **caractérisé en ce que** le cylindre hydraulique (40), en particulier son tube cylindrique (42) et/ou sa base de cylindre (44), est fixé sur l'unité de tête de colonne (12).

14. Dispositif (10) selon l'une quelconque des revendications précédentes, **caractérisé en ce que** le cylindre hydraulique (40) comprend une chemise cylindrique (42), une base de cylindre (44), un piston (46) mobile à l'intérieur de la chemise cylindrique (42) et une tige de piston (48) fixée sur le piston (46), et que le dispositif d'engrenage (60) est disposé sur l'extrémité de la tige de piston (48) opposée au piston (46).

15. Procédé de montage d'un dispositif (10) selon l'une quelconque des revendications 1 à 14,
dans lequel le cylindre hydraulique (40) est monté en insérant en premier une base de cylindre (44) dans une chemise cylindrique (42), jusqu'à ce qu'il soit intégralement logé dans une telle chemise,
puis l'on introduit un piston (46), une tige de piston (48) et des éléments d'étanchéité (50) dans le tube cylindrique (42),
l'unité ainsi obtenue étant utilisée dans des éléments de logement (41) d'un cadre (16) de l'unité de tête de colonne (12),
puis la tête de colonne (47) du tube cylindrique (42), de laquelle la tige de piston (48) dépasse, est pressurisée par une pression, de telle sorte qu'une partie de la base de cylindre (44) est pressée hors du tube cylindrique (42) dans une cavité de logement (54) du cadre (16),
puis une conduite d'huile est raccordée au côté de la base du cylindre hydraulique (40),
et dans lequel l'on fixe le dispositif d'engrenage (60) sur l'extrémité de la tige de piston (48) opposée à a chemise cylindrique (42).
